# EUROPÄISCHE PATENTSCHRIFT

(11) **EP 1 897 505 B1**
(45) Veröffentlichungstag und Bekanntmachung des Hinweises auf die Patenterteilung: **03.08.2011**
(21) Anmeldenummer: 07016140.1
(22) Anmeldetag: 17.08.2007
(51) Int. Cl.: A61B 17/28

(54) **Medizinisches Instrument**
Medical instrument
Instrument médical

(30) Priorität: 09.09.2006 DE 102006042889
(43) Veröffentlichungstag der Anmeldung: 12.03.2008
(73) Patentinhaber: CARDIOMEDICAL GmbH, 30855 Langenhagen (DE)
(72) Erfinder: Wiedenbein, Wolfgang, 30926 Seelze (DE)

(56) Entgegenhaltungen:
- WO-A-2006/078661
- DE-A1- 10 314 072
- DE-A1-102004 025 041
- DE-A1-102004 033 290

## Beschreibung

### Technisches Gebiet

Die vorliegende Erfindung bezieht sich allgemein auf das Gebiet der Medizin in der Diagnostik und Chirurgie. Im diagnostischen und chirurgischen Bereich werden, unter Anwendung der Endoskopie, chirurgische Instrumente, Vorrichtungen oder Verfahren, um das Innere von lebenden Organismen zu untersuchen und/oder für operative Eingriffe zu nutzen, eingesetzt. Chirurgische Instrumente für die Endoskopie sind hinreichend bekannt. Endoskopische Instrumente finden beispielsweise auch in der laparoskopischen Chirurgie Anwendung, bei der, mit Hilfe eines optischen Instruments, Eingriffe innerhalb der Bauchhöhle vorgenommen werden, insbesondere in der minimalinvasiven Chirurgie. Hierbei werden durch kleine Körperöffnungen Trokare in den Körper eines Patienten eingeführt. Durch die Öffnungen werden anschließend das endoskopische Instrument zur Bildgebung und/oder flexible Greifoder Schneidwerkzeuge zwecks Gewinnung von Gewebeproben (Biopsien), durchgesteckt. Auch können andere chirurgische Instrumente durch die Öffnung im Trokar in den Hohlraum eingeführt werden. Auf diese Weise kann beispielsweise ein dorsaler oder auch ein ventraler Zugang zur Wirbelsäule geschaffen werden, um anschließend mit Hilfe der chirurgischen Instrumente minimalinvasiv einen Eingriff vorzunehmen. Hierzu ist, nur um beispielsweise einige zu nennen, am distalen Ende des Instrumentenschaftes ein Arbeitselement, , ein scharfer Löffel, ein Stößel, ein Schneidwerkzeug oder ein Explorationshäkchen angeordnet.

Während der Operation kommen, bei eingeführtem Trokar, unterschiedliche endoskopische Instrumente, die jeweils ein spezielles Arbeitselement tragen, zum Einsatz. Zur Bedienung des am distalen Ende des Instrumentenschaftes angeordneten Arbeitselements enthält das endoskopische Instrument am proximalen Ende des Instrumentenschaftes ein Bedienungselement. Das Bedienungselement beeinflusst maßgebend das Handling des chirurgischen Instruments.

Nach der Operation des Patienten sind die benutzten chirurgischen Instrumente durch Körperflüssigkeiten kontaminiert und müssen daher gereinigt werden. Zur Reinigung können die meisten chirurgischen Instrumente in ihre Bestandteile zerlegt werden, wobei die Reinigung des Innenteiles des röhrenförmigen Instrumentenschaftes eine hohe Herausforderung an den Reinigungsprozess stellt. Zur Zerlegung des chirurgischen Instruments kann das in dem röhrenförmigen Element verschiebbar geführte Zug- und/oder Druckelement, welches am distalen Ende ein Arbeitsmittel betätigt und am proximalen Ende über ein Koppelelement verfügt und lösbar mit dem beweglichen Bedienelement verbunden ist, aus dem Instrumentenschaft gezogen werden. Der Koppelstelle kommt daher, einerseits zur Übertragung der Zug- und Druckkräfte von der Betätigungsstange des Bedienelements auf das Zug- und Druckelement im röhrenförmigen Instrumentenschaft, und andererseits in Hinsicht auf ein einfaches Handling bei der Trennung des Instrumentenschaftes vom Bedienelement, besondere Bedeutung zu.

Die Erfindung betrifft somit ein medizinisches Instrument, insbesondere ein chirurgisches Instrument zur Verwendung in der minimalinvasiven Chirurgie, mit einem röhrenförmigen Element, einem Arbeitselement und einem Bedienelement, welches lösbar am proximalen Ende des röhrenförmigen Elements angeordnet ist und ferner mit einem in dem röhrenförmigen Element verschiebbar geführten Zug- und Druckelement, dessen vorderes Ende ein Arbeitselement betätigt und dessen rückwärtiges Ende über ein Koppelelement lösbar mit dem beweglichen Bedienelement verbunden ist.

### Hintergrund im Stand der Technik

Solche medizinischen Instrumente, insbesondere chirurgische Instrumente in verschiedenen Bauarten und Ausführungen, haben sich in der Endoskopie, insbesondere in der Laparoskopie bei der Untersuchung und beim minimalinvasiven Einsatz am Patienten, vielfach bewährt. Dem Bedienelement solcher chirurgischen Instrumente kommt daher besondere Bedeutung zu.

Wie aus dem Stand der Technik bekannt, kann das Bedienelement für die chirurgischen Instrumente aus verschiedenartig ausgeführten Griffen, wie beispielsweise einem so genannten Pistolengriff, aus einem Zweiring- Griff, ähnlich dem Griff einer Schere oder Axialgriff, bestehen, wobei in vielen Fällen die Griffe mit einem Verriegelungselement ausgestattet sind. Ein Beispiel eines Bedienelements mit Scherengriff und Sperre ist der DE 201 21 753 U1 zu entnehmen. Das Bedienelement besteht aus einem Griff mit zwei gegeneinander bewegbaren Griffteilen und mit einer lösbaren Sperre, welche die Griffteile in ihrer gegenseitigen Position arretiert, wobei die Sperre ein an einem der Griffteile angebrachtes Schiebeglied aufweist, welches in einem an dem anderen Griffteil angebrachten Klemmgehäuse axial verschiebbar ist, dass das Schiebeglied durch ein federbelastetes Klemmelement in dem Klemmgehäuse gegen eine Verschiebung klemmbar ist und dass das Klemmelement gegen die Federkraft aus seiner Klemmstellung bewegbar ist.

Die Ausfiihrungsform eines solchen Bedienelements ist in einigen Operations-Anwendungsfällen in der Endoskopie-Chirurgie in den Außenabmessungen zu groß und zu unhandlich und der Bedienungswinkel, d.h. die Stellung des abgewinkelten Bedienelements zum Instrumentenschaft, ist ungünstig in der Handhabung. Das Verriegelungselement der Sperre besteht aus einer Vielzahl von Einzelteilen, die ebenfalls relativ groß und sperrig sind. Des weiteren ist das dargestellte Bedienelement mit angeschlossenem Instrumentenschaft zur Reinigung desselben wegen fehlendem Spülanschluss nicht geeignet. Ein Kupplungsanschluss zwischen Instrumentenschaft und Griffteilen fehlt ebenfalls.

Die vorgenannten Angaben treffen auch auf die in der DE 298 04 860 U1 und der DE 697 24 040 T2 offenbarten chirurgischen Instrumente mit deren Griffteilen zu, wobei es sich bei beiden Griffteilen um Scherengriffe handelt, welche auch keinen Verriegelungsmechanismus aufweisen. Ebenfalls fehlt eine Kupplung zwischen dem Instrumentenschaft und den Griffteilen. Ein Reinigungsanschluss für Flüssigkeiten ist auch nicht vorhanden.

Daher schlägt die DE 10 2004 009 200 A1 zur Reinigung des Instrumentenschaftes eines chirurgischen Instruments vor, welches mit einem Rundstangenschaft für den endoskopischen Einsatz in pistolenartiger Bauart mit angeordneten Betätigungsgriff für eine am Instrumentenschaft befindliche Maulmechanik ausgebildet ist, die Betätigungsstange griffseitig lösbar aus der starren Schaftstange zum Zwecke der Reinigung ausschwenkbar zu gestalten. Der Nachteil dieser Ausführungsform besteht darin, dass der Instrumentenschaft zur Reinigung zerlegt werden muss und ein Aus- und Einkoppeln des Instrumentenschaftes mit dem Bedienelement umständlich ist. Ein Verriegelungsmechanismus in den Griffteilen ist ebenfalls nicht vorhanden.

Weitere chirurgische Instrumente mit pistolenartigem Griff sind aus der DE 695 28 416 T2 und der DE 10 2004 031 928 A1, die in der Endoskopie- Chirurgie Anwendung finden, ersichtlich. Auch hier sind die Griffteile nicht mit Verriegelungsmechanismen ausgestattet. Des weiteren enthält die Verbindungsstelle zwischen dem Rohrschaft bzw. Instrumentenschaft und den Griffteilen kein Kupplungselement. An der Verbindungsstelle fehlt ebenfalls ein Reinigungsanschluss, der das Reinigen des Instrumentenschaftes ermöglicht. Des weiteren fehlen Verriegelungselemente bzw. lösbare Sperren an den beweglichen Griffen.

Aus dem Als naheliegenster Stand der Technik bei chirurgischen Instrumenten mit einem pistolenartigen Griff als Bedienungselement ist die in der DE 10 2004 025 041 A1 offenbarte Laparoskopie- Zange zu nennen. Das Laparoskopieinstrument ist mit einem festen Griffstück mit dem über ein Gelenk bewegliches Griffstück verbunden. Am festen Griffstück befindet sich ein Anschlussnippel zur Verbindung mit einem Schlauch für die Zuführung eines Reinigungsmediums. Zwischen dem beweglichen Griffstück und der Betätigungsstange befindet sich ein Zwischenglied, welches aus einem Schieber besteht, der eine Koppelstange enthält, an deren Ende ein Kugelkopf angeordnet ist. Der Nachteil dieser Ausführungsform ist, dass das Bedienelement relativ groß ist, keine lösbare Sperre für die Griffstücke besitzt und dass das Koppelelement aus einer Vielzahl von Einzelteilen gebildet wird. Ebenfalls nachtteilig wirkt sich in der Handhabung des chirurgischen Instruments in einigen Operations- Anwendungsfällen der Bedienungswinkel, d.h. die Stellung des abgewinkelten Bedienelements zum Instrumentenschaft, aus. Des weiteren kann die Verschwenkung der beweglichen Branche am Griffteil eine negative Bewegung am zu operierenden Organ zur Folge haben, dieses gilt für alle großen Ausführungen von Handgriffen.

Aus dem Stand der Technik wird noch die DE 10 2004 033 290 A1 zitiert, welche ein chirurgisches Rohrschaftinstrument mit axialen Federgriff offenbart. Dieses Instrument gilt als Basis bzw. als. Ausgang der Entwicklung. Aufgabe der Entwicklung war die Nachteile dieses Instrumentes zu beseitigen. Dieses Instrument enthält keinen Verriegelungsmechanismus in den axialen Federgriffen, keine Kupplung zwischen dem Instrumentenschaft und keinen Reinigungsanschluss an der Verbindungsstelle zwischen dem Schaft und den Griffen.

Stellvertretend für die vielen chirurgischen Instrumente mit axialen Bedienungselement bzw. Handgriff, sei noch die DE 91 14674.7 U1 genannt, die zwar eine lösbare Sperre für die Handgriffe besitzt, aber keine geeignete Koppelstelle zur Aufnahme von unterschiedlichen Instrumentenschäften besitzt. Zur Reinigung ist das Instrument nicht zerlegbar und besitzt am Handgriff keinen Anschluss für Reinigungsflüssigkeiten.

Eine andere Aüsfiihrungsform eines chirurgischen Instruments mit Axialgriff und Verriegelungsknopf ist der EP 0 820 725 B1 zu entnehmen. Dieses Instrument enthält keine Koppelstelle und ist somit nicht zwischen dem Instrumentenschaft und dem Bedienelement zerlegbar. Ein Anschluss für Reinigungsflüssigkeiten am Axialgriff fehlt ebenfalls.

Bei den chirurgischen Instrumenten mit axialem Griff ist auch die EP 0 327 410 B1 aus dem Stand der Technik zu nennen. Bei diesem Instrument, welches als multifunktionelles Instrument in der Laparoskopie eingesetzt wird, ist eine Zerlegbarkeit zwischen Instrumentenschaft und Bedienelement gegeben. Die Zerlegung kann aufgrund eines Bajonettverschlusses zwischen der Kanüle und dem Handgriff erfolgen, wobei an der Kanüle ein spezielles Anschlussstück vorhanden ist, welches auch einen Anschluss für eine Spülleitung aufweist. Der Handgriff wird für verschiedene Funktionen elektrisch durch eine Mehrzahl von Steuermitteln bedient. Diese spezielle Ausführung beinhaltet verschiedene Nachteile. Der wichtigste Nachteil besteht darin, dass der Handgriff des Instrumentes nicht kompatibel zu anderen Instrumenten- Schäften ist und das durch den Anschluss einer elektrischen Leitung am Handgriff, das Handling des Instrumentes, für den Chirurgen erschwert wird. Bei dem vorgenannten Instrument handelt es sich auch nicht um ein chirurgisches Instrument mit axial beweglichen Handgriffen.

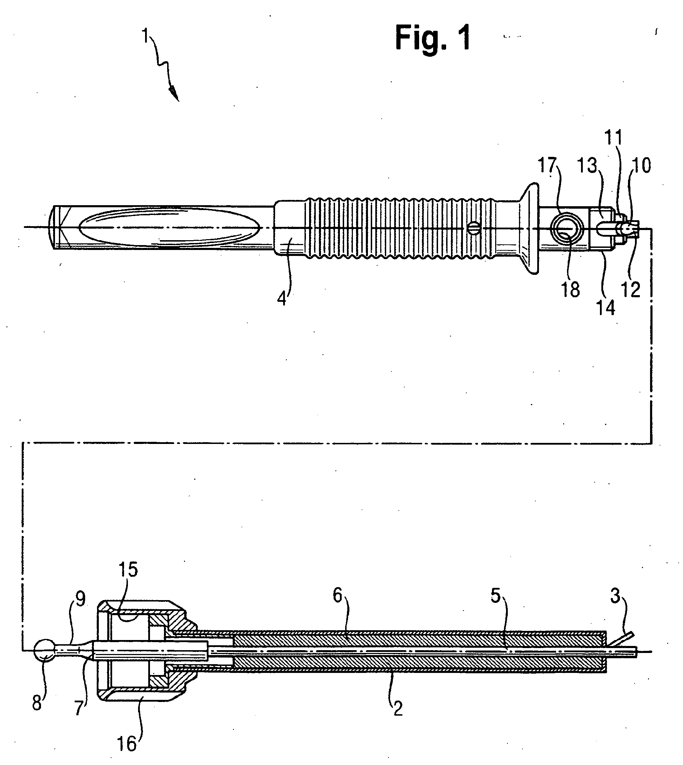

Zum naheliegenden Stand der Technik könnte auch der aus der DE 103 14 072 A1 offenbarte Gegenstand einer Umlaufsperre in einem chirurgischen Instrument gehören. Bei diesem chirurgischen Instrument handelt es sich um ein Instrument, welches als Bedienelement einen zangenartigen Griff ausweist, der mit einer großen Öffnung am proximalen Ende ausgestattet ist und zur Bedienung über eine Biegefeder verfügt. Gemäß der Figur 1 und der Figurenbeschreibung besteht das Bedienelement aus einem bewegbaren Griffteil und einem feststehenden Griffteil. Dieses Bedienelement muss aufgrund der Größe und Ausführung der Griffteile, zur Betätigung mit der ganzen Hand erfasst werden. Aufgabe der Erfindung ist es aber, die Ausführungsform eines chirurgischen Rohrschaftinstruments mit axialen Federgriff, wie in der DE 10 2004 033 290 A1 zitiert, anzuwenden, wobei das Bedienelement am proximalen Ende keine Öffnung enthält und das Bedienelement, bzw. die Griffteile mit zwei Fingern zu betätigen ist. Das feststehende und das bewegbare Griffteil enthalten zwar eine Umlaufsperre, welche in der offenbarten Ausführungsform aber nicht für den Einsatz in einem axialen Bedienelement mit zwei beweglichen Griffteilen, gemäß der DE 10 2004 033 290 A1, einsetzbar ist. Der im feststehenden Griffteil angeordnete Rastkörper, bzw. Haltearm, ausgestattet mit einer zylindrischen Feder, deren Dreh- und Befestigungspunkt oberhalb der zylindrischen Feder angeordnet ist, lässt sich auch nicht in naheliegender weise in ein bewegliches axiales Bedienelement mit Federgriff integrieren. Auch der im beweglichen Griffteil angeordnete Einsatz mit w- förmiger Vertiefung, wobei die w- förmige Vertiefung Steuerkurven enthält, lässt sich nicht in einen axialen Federgriff integrieren. Der Nachteil, der in der DE 103 14 072 A1 offenbarten Umlaufsperre zur gegenseitigen Fixierung der Griffteile besteht darin, das in den Griffteilen große Ausnehmungen (Öffnungen) benötigt werden, welche in den der DE 10 2004 033 290 A1 zitierten axialen Griffteilen aber nicht vorhanden sind.

Der Erfindung liegt daher die Aufgabe zugrunde, ein medizinisches Instrument für die minimalinvasive Chirurgie der eingangs genannten Art zu schaffen, welche die vorgenannten Nachteile und Unzulänglichkeiten der bekannten Anordnungen vermeidet und eine technische Lösung anzugeben, die es ermöglicht, ein kostengünstigeres, mit einfacher Funktionsgeometrie ausgestattetes chirurgisches Instrument mit axialen Bedienelement für die Endoskopie mit erhöhten Ansprüchen zu schaffen, welches eine Bauform aufweist, die ein einfaches Ein- und Auskoppeln von verschiedenen Ausführungsformen von Instrumenten- Schäften an der Koppelstelle ermöglicht und zur einfacheren Reinigung geeignet ist, sowie einen integrierten und bedienungsunterstützenden Verriegelungsmechanismus in den Griffstücken des Bedienelements zur Verfügung zu stellen.

Erfindungsgemäß wird dieses Problem durch die Merkmale des Patentanspruchs 1 gelöst. Vorteilhafte Ausgestaltungen und Weiterbildungen der Erfindung ergeben sich aus den nachfolgenden Unteransprüchen und den nachstehenden Beschreibungen.

### Beschreibung der Erfindung

Um ein mit diesen Merkmalen der vorliegenden Erfindung ausgestattetes medizinisches Instrument, insbesondere chirurgisches Instrument zur Verwendung in der minimalinvasiven Chirurgie für chirurgische Anwendungen herzustellen wird erfindungsgemäß vorgeschlagen, das chirurgische Instrument so zu gestalten, dass dem Chirurgen ein ergonomisches Bedienelement zur Verfügung steht, das die Handhabung wesentlich erleichtert und die Präzision des Eingriffs verbessert. Um dieses optimal zu erreichen, ist es notwendig, den durch den Chirurgen vorzunehmenden Eingriff am Patienten vorher zu analysieren.

Bei der Anwendung der endoskopischen Chirurgie wird ein Trokar, eine spitze Durchstechvorrichtung, in einen Körper eingebracht, wobei der Trokar von einer Kanüle umgeben ist. Nachdem der Trokar das Durchstechen, beispielsweise der Bauchwand durchgeführt hat, wird er entfernt, wobei die Kanüle im Körper verbleibt. Häufig werden mehrere Öffnungen durch den Trokar im Körper erzeugt, so dass in der einen Kanüle ein endoskopisches Instrument, in einer anderen Kanüle eine Beobachtungseinrichtung und in einer weiteren Kanüle eine Faseroptik zur Beleuchtung des chirurgischen Arbeitsfeldes platziert werden können. Es besteht aber auch die Möglichkeit, durch ein und dieselbe Kanüle unterschiedliche chirurgische Instrumente einzuschieben. Durch das Ein- und Ausschieben eines chirurgischen Instruments kann die Operationsstelle gewebetraumatisch reagieren.

Das endoskopische chirurgische Verfahren bringt daher gewisse Probleme mit sich. Da der aktive Teil des Instruments, der sich im Körperinneren befindet, vom manipulierten Teil des Instruments, der außerhalb des Körpers ist, weiter entfernt liegt, ist jede geringfügige Bewegung am manipulierten Teil vergrößert, wenn sie den aktiven Teil erreicht. Daher muss die Hand des Chirurgen während des endoskopischen Eingriffs viel sicherer sein, als wenn er denselben Eingriff im Rahmen der offenen Chirurgie durchführt. Eine weitere Schwierigkeit besteht darin, dass der den Eingriff durchführende Chirurg das Feld, in dem er operiert, nicht unmittelbar einsehen kann, sondern dass er dieses auf einem Videodisplay beobachtet, und die Instrumente auf Basis dessen, was er auf dem Videodisplay sieht, manipuliert. Ferner kann der Chirurg nicht mehr das Gewebe mit der Hand fühlen, um dessen Dicke, Beschaffenheit und Struktur etc. zu bestimmen, und der Zugriff der chirurgischen Instrumente ist begrenzt. Und schließlich ist es schwierig, den Kopf des Instrumentes zu manipulieren, nachdem er in der Kanüle platziert worden ist. Sein Bewegungsbereich ist begrenzt. Angesichts dieser schwierigen Vorgaben ist bei der Konstruktion des erfindungsgemäßen chirurgischen Instruments für die Endoskopie-Chirurgie auch versucht worden, die Kraft zu reduzieren die erforderlich ist, um die Arbeitselemente zu betätigen, und dem Chirurgen eine größere Kontrolle über das Instrument zu ermöglichen. Weil die chirurgische Technik heute den direkten kontrollierten Zugang zu einer Vielzahl von inneren Organen ohne offene chirurgische Eingriffe ermöglicht, kommt den beim Eingriff verwendeten chirurgischen Instrumenten eine besondere Bedeutung zu. Die Zugänge zu den Operationsstellen können einerseits durch natürliche Körperöffnungen vorgegeben sein oder andererseits mittels kleiner Inzisionen geschaffen werden, die wie zuvor beschrieben, mit einem haltenden Instrument eine Trokares offen gehalten werden. Es besteht aber auch die Möglichkeit als haltendes Instrument für geschaffene Körpereingänge Spreizer oder Retraktoren einzusetzen, durch die das chirurgische Instrument mit fassenden oder schneidenden Arbeitsmittel eingreifen kann.

Daher besteht die erste besondere Aufgabe der Erfindung darin, dem Chirurgen ein ergonomisch gestaltetes chirurgisches Instrument zur Verfügung zu stellen, welches die Handhabung wesentlich erleichtert und die Präzision des Eingriffs verbessert. D.h. den Einsatz großer Handgriffe, wie beispielsweise aus dem Stand der Technik bei Scheren griffen und Pistolengriffen bekannt, zu vermeiden. Der Einsatz eines solchen erfindungsgemäßen chirurgischen Instruments ist für die verschiedensten Eingriffe konzipiert, insbesondere für den Einsatz mit Retraktor, der beispielsweise zwischen den Rippen angeordnet den Zugang zum Cardia ermöglicht.

Ein erfindungsgemäßes chirurgisches Instrument, welches die vorgenannten Erfordernisse erfüllt, umfasst die Merkmale von Anspruch 1.

Um die Handhabung und die Präzision des chirurgischen Instruments zu verbessern, ist die technische Ausgestaltung des Verriegelungssystems derart gestaltet, dass das System selbstständig Funktionen die sonst der Benutzer durchgeführt hat, zu übernehmen.

Dazu ist der Verriegelungsmechanismus im Bedienelement integriert und ver- oder entriegelt automatisch durch die Bewegung der Griffstücke das Bedienelement. Dabei umfasst der Verriegelungsmechanismus folgende Elemente, eine Riegelfeder, einen Riegelarm, eine Riegelraste und einen Verriegelungsstift, wobei die Riegelfeder, der Riegelarm und die Riegelraste miteinander eine Funktionseinheit bilden. Die Riegelfeder ist dazu zwischen einer Griffschale und einem der gefederten Schenkel angeordnet, wobei der an der Riegelfeder angeordnete Riegelarm einen Befestigungsstift aufweist der einen Riegeldrehpunkt bildet, um den der Riegelarm drehbar gelagert ist. Zur Aufnahme des Riegelarms sind die gefederten Schenkel mit einer Ausnehmung und der Grundkörper mit einem Durchbruch versehen. Der Riegelarm kann somit um den Riegeldrehpunkt in der Ausnehmung des einen gefederten Schenkels und dem Durchbruch im Grundkörper nach rechts und links Ausschwenken. Die Ausschwenkung des Riegelarms erfolgt durch die Riegelraste, wenn diese am starren Verriegelungsstift, der in der Ausnehmung des anderen gefederten Schenkels angeordnet ist, entlang gleitet. Zum entlang Gleiten am Verriegelungsstift ist die Riegelraste mit Gleitflächen ausgestattet ist. Die Gleitflächen der Riegelraste bilden für den Verriegelungsstift einen Einrastweg, eine Raststellung und einen Ausrastweg, wobei der Verriegelungsstift in der Position der Raststellung das Bedienelement verriegelt. In der verriegelten Stellung sind die Griffstücke am Bedienelement verschlossen. D.h. die erste Funktion des Verriegelungsmechanismus besteht darin, automatisch beim Schließen der Griffstücke, das Bedienelement zu verriegeln. Der Verriegelungsmechanismus greift aber nur, wenn die Griffstücke bis zum Anschlag zusammen gedrückt werden. Die zweite Funktion besteht im Entriegelungsvorgang. Zum Entriegel des Bedienelements brauchen die Griffschalen nur mit einer Druckkraft, erzeugt durch Zeigefinger und Daumen, beaufschlagt werden, wodurch sich die Griffstücke des Bedienelements öffnen. Die Funktionen Einrasten wie Ausrasten erfolgen dadurch, dass der Verriegelungsmechanismus bzw. die Riegelraste seitlich verschoben wird, wenn der gefederte und seitlich verschwenkbare Riegelarm durch Schließen des Axial handgriffes, gegen den starren Verriegelungsstift gedrückt wird. Zum Verriegeln und Entriegel sind somit keine zusätzlichen Handbewegungen des Benutzers notwendig, wodurch das Handling und die Präzision beim Eingriff sicherer werden. Um das Handling optimal zu gestalten sind die Griffschalen ergonomisch gestaltet und an der Benutzeroberfläche strukturiert.

Eine zweite besondere Aufgabe besteht darin, ein Instrument zur Verfugung zu stellen, welches leicht zu Reinigen und zu Sterilisieren ist und zwar im Bezug auf die Teile die in direkten oder indirekten Kontakt mit organischen Gewebe und Köperflüssigkeiten gelangen und um eine den medizinischen Anforderungen der EU-Richtlinie entsprechende Reinigung und Sterilisation am chirurgischen Instruments durchführen zu können und somit von Partikeln zu reinigen.

Diese Aufgabe wird erfindungsgemäß dadurch gelöst, das an dem Bedienelement, welches mit einer Instrumentenschaftaufnahme ausgestattet ist, mit einem Anschlussnippel auszubilden, an dem eine Spülleitung angeschlossen werden kann. Der erfindungsgemäße Anschlussnippel angeordnet am Instrumentenschaft besitzt den Vorteil, das während der Operation für verschiedene Arbeitsschritte bereits am Operationstisch eine Spülung des Instrumentenschaftes zwischen den verschiedenen Arbeitsschritten erfolgen kann, wodurch die Gefahr der Kontaminierung der OP-Stelle bzw. eine Kontamination der inneren Organe reduziert wird. Der Anschlussnippel ist derart gestaltet, das verschiedene Anschlussmöglichkeiten daran bestehen. Beispielsweise kann eine Leitung für Flüssigkeiten angeschlossen werden, wobei die Flüssigkeiten aus verschiedenen flüssigen Medien bestehen können, vorzugsweise einer Reinigungsflüssigkeit. Am OP-Tisch können zum Anschluss vorzugsweise Spritzen benutzt werden. Der Anschlussnippel erlaubt somit das hindurch laufen von Flüssigkeit durch den Instrumentenschaft. Auch besteht die Möglichkeit den Anschlussnippel für andere Flüssigkeiten zu benutzen.

Der Zerlegbarkeit von chirurgischen Instrumenten zum Zwecke der Reinigung und Sterilisation kommt daher auch eine besondere Bedeutung zu. Die dritte besondere Aufgabe besteht also darin, ein chirurgisches Instrument zu schaffen, welches ein Bedienelement enthält, das einerseits zum Anschluss einer Vielzahl von unterschiedlichen Instrumentenschäften und somit von unterschiedlichen Arbeitselementen geeignet ist und andererseits eine Anschlussstelle zur Verfügung stellt, die ein schnelles und einfaches Wechseln von Instrumentenschäften ermöglicht. Um das erfindungsgemäße chirurgische Instrument während einer Operation für verschiedene Arbeitsschritte verwenden zu können, ist bei einer besonders bevorzugten Ausgestaltung vorgesehen, das Bedienungselement mit dem Instrumentenschaft lösbar zu verbinden. Auf diese Weise können verschiedene Arbeitselemente mit dem Instrumentenschaft am Bedienelement verbunden werden, beispielsweise einem Explorationshäkchen, Disektoren, scharfe Löffel, Osteotome und Stößel usw. nur um einige zu nennen, wobei es nicht erforderlich ist das gesamte chirurgische Instrument auszutauschen, sondern nur den Instrumentenschaft. Das Bedienelement bleibt das Gleiche, was die Handhabung nochmals erleichtert. Erfindungsgemäß wird diese Aufgabe dadurch gelöst, das die in Instrumentenschaftaufnahme angeordnete Kupplung eine Öffnung aufweist, die geeignet ist, Instrumentenschäfte die als Koppelelement einen Kugelkopf aufweisen, aufzunehmen. Zum schnellen und einfachen Wechsel der unterschiedlichen Instrumentenschäfte bzw. Arbeitsmittel, die mit einem Kugelkopf ausgestattet sind, ist die erfindungsgemäße Öffnung, die einem Schlüsselloch gleicht, in der Kupplung mit einer Einführschräge ausgebildet. Diese Besonderheit ermöglicht eine einfachere Montage und Demontage.

Ein Ausführungsbeispiel der Erfindung ist in den Zeichnungen rein schematisch dargestellt und wird nachfolgend näher beschrieben. Es zeigt
- Figur 1: in perspektivischer Darstellung eine Gesamtansicht des erfindungsgemäßen chirurgischen Instruments und
- Figur 2: in perspektivischer Seitenansicht ein erfindungsgemäßes Bedienungselement mit geöffneter Griffstellung und
- Figur 3: eine seitliche Darstellung des Bedienelements im Schnitt mit angeschlossenem Instrumentenschaft

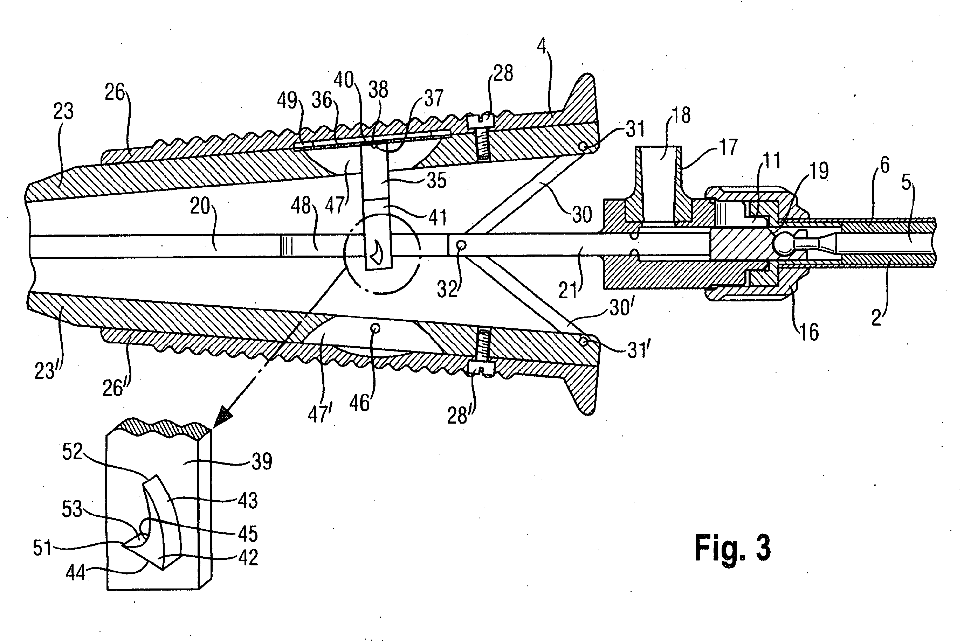

Das in Fig.1 in Draufsicht dargestellte chirurgische Instrument 1 besteht im Wesentlichen aus einem axialen Bedienelement 4, einem darin integrierten Verrieglungsmechanismus 35 (siehe Fig.3), einem daran angeordneten röhrenförmigen Element 2 (im Schnitt), nachstehend als Instrumentenschaft bezeichnet, an dessen distalen Ende ein Arbeitselement 3 herausschaut. In den Zeichnungen ist der Begriff "proximal", wie herkömmlich, auf das Ende des Instrumentenschaftes 2 bezogen, das näher zu dem Bediener liegt, während der Begriff "distal" sich auf das Ende beziehen wird, das von dem Bediener weiter entfernt ist. Das Arbeitselement 3 ist am vorderen Ende des Zug- und Druckelements 5 angeordnet. Das verschiebbare Zug- und Druckelement 5, welches von einem durch ein im röhrenförmigen Element 2 geführtes Schaftrohr 6 umgeben ist, besitzt am rückwärtigen Ende ein Koppelelement 7. Das Koppelelement 7 am Zug- und Druckelement 5 besteht aus einer Kugel 8, die in eine Öffnung 10 einer Kupplung 11, die von einer Instrumentenschaftaufhahme 13 umgeben ist, eingreift. Die Instrumentenschaftaufnahme 13 besitzt ein Außengewinde 14 zur Befestigung des korrespondierenden Innengewinde 15, der am proximalen Ende des Instrumentenschafts 2 angeordneten Überwurfmutter 16, wodurch der Instrumentenschaft 2 am Bedienelement 4 lösbar befestigt ist. Des weiteren ist an der Instrumentenschaftaufnahme 13 ein Nippel 17 mit Bohrung 18 zur Aufnahme einer Reinigungsleitung (nicht dargestellt) angeordnet. Die Reinigungsleitung am Anschlussnippel 17 dient der Zuführung eines Mediums, etwa einer Spülflüssigkeit o.ä. zur Operationsstelle.

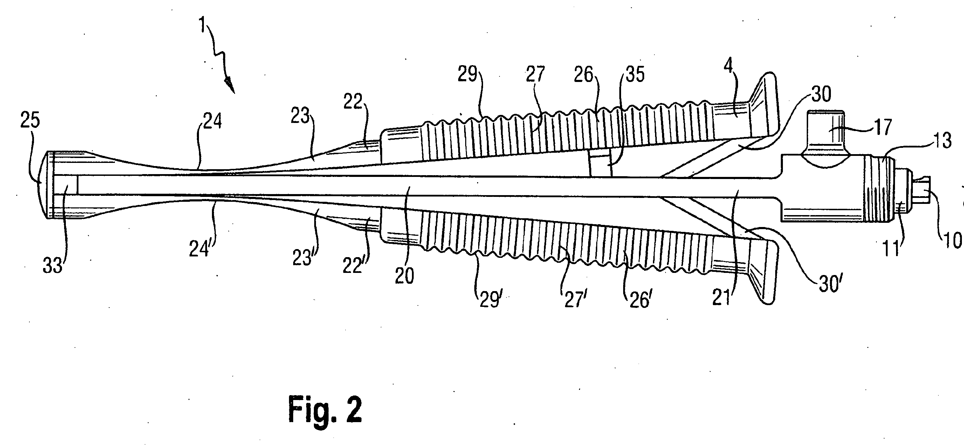

Die Instrumentenschaftaufnahme 13 ist Bestandteil des axialen Bedienelementes 4, welches anhand der Fig.2 und Fig.3 in seiner Funktion, sowie die Kopplung der Betätigungsstange 21 mit den beweglichen Griffstücken 27,27' einerseits und die Kopplung mit dem verschiebbaren Zugund Druckelement 5 andererseits, näher erläutert werden. Die Funktion des integrierten Verriegelungsmechanismus 35 in Verbindung mit den Griffstücken 27,27' und dem Grundkörper 20 wird in Fig.3 aufgezeigt. Eine erfindungsgemäße Ausführungsform des axialen Bedienelements 4 mit geöffneter Stellung der beiden symmetrisch angeordneten Griffstücke 27,27' in seitlicher Ansicht zeigt die Fig.2 und in seitlicher Schnittdarstellung die Fig.3, wobei mit gleichen Bezugszeichen identische Teile wie in Fig.1 bezeichnet werden. Das axiale Bedienelement 4 hat die allgemeine Form eines lang gestreckten zylindrischen Körpers ohne Neigung zur Achse des Instrumentenschaftes (siehe Fig.1), wobei das axiale Bedienelement 4 aus einem lang gestrecktem Grundkörper 20 besteht, der beweglich angeordnet ist. Der bewegliche Grundkörper 20 bildet am distalen Ende eine Betätigungsstange 21, die in die Instrumentenschaftaufnahme 13 eintaucht und mit der Kupplung 11 verbunden ist. Die Kupplung 11 der Instrumentschaftaufnahme 13 enthält eine Öffnung 10 zur Aufnahme des Koppelelements 7, bzw. die geeignet ist, einen Kugelkopf eines Zug- und Druckelements 5 aufzunehmen. Die Form der Öffnung 10 der Kupplung 11 ist derart gestaltet, das diese ähnlich der Form eines Schlüssellochs 12, mit einer Einführschräge 19, entspricht. Das Schlüsselloch 12 mit der Schräge 19 dient zur leichteren Montage und Demontage bzw. zur Aufnahme des Koppelelements 7, welches aus einer Kugel 8 und der Verjüngung des Schaftes 9 des Zug- und Druckelements 5 gebildet wird, wobei der verjüngte Schaft 9 und die daran angeordnete Kugel 8 in der Ausführungsform der Form einer Anhängerkupplung von Fahrzeugen ähnlich ist. Die Kupplung 11 stellt somit eine schlüssige Verbindung zwischen dem Bedienelement 4 und dem Arbeitselement 3 sicher.
Der bewegliche Grundkörper 20 wird von einem Element 22 umschlossen, welches in Form und Funktion einer Pinzette nachempfunden ist. Der erfindungsgemäße Unterschied besteht darin, dass die Pinzettenarme 23,23' auf der vom Grundkörper 20 abgewandten Seite, eine Verjüngung 24,24' aufweisen, die fertigungstechnisch so hergestellt sind, sodass dadurch eine Federspannung in den Pinzettenarmen 23,23' entsteht, wodurch diese die Bestrebung haben sich zu öffnen. Die Pinzettenarme 23,23'bilden somit gefederte Schenkel 23,23'. Dieser Effekt wird beim erfindungsgemäßen Verriegelungsmechanismus 35 benötigt und in Fig.3 näher beschrieben. Am proximalen Ende des Grundkörpers 20 befindet sich der Scheitelpunkt 25 des Pinzettenelements 22. Die beiden gefederten Schenkel 23,23' des Pinzettenelements 22 sind des weiteren Träger der Griffschalen 26,26' und bilden mit diesen die beweglichen Griffstücke 27,27', die das Bedienelement 4 in etwa halbkreisförmig umschließen. Die Griffschalen 26',26' sind durch Befestigungsmittel 28,28', wie Schrauben, Nieten oder Kleben usw., mit den gefederten Schenkeln 23,23' fest verbunden und an der Benutzeroberfläche 29,29' strukturiert. Die Strukturierung entspricht den ergonomischen Anforderungen einer Hand, wobei die Oberflächenstruktur der Griffschalen 26,26' in der Fig.2 wellenförmig ausgebildet ist. Andere Oberflächenstrukturen sind ebenfalls herstellbar. Am distalen Ende der beweglichen Griffstücke 27,27' sind gelenkartig Bauteile 30,30' in Form von Hebeln, an der Unterseite der gefederten Schenkel 23,23' der dem Grundgehäuse 20 zugewandten Seite, angeschlossen, wobei das Bauteil 30,30' einerseits um einen Befestigungsstift 31,31' im Griffstück 27,27' drehbar gelagert und andererseits um einen Befestigungsstift 32 im Grundkörper 20 drehbar angeordnet ist. Beim Schließen der symmetrisch sich gegenüberliegenden Griffstücke 27,27' aus der geöffneten Position von Fig.2 in die geschlossene Position der Fig.1 wird die Betätigungsstange 21 des Grundkörpers 20 durch die an gelenkten Hebel 30,30' in axialer Richtung verschoben. Die Verschiebung des Grundkörpers 20 hat zur Folge, dass das über die Betätigungsstange 21 und dem Zug- und Druckelement 5, am Ende des distalen Instrumentenschaftes 2, angeschossene Arbeitselement 3 ebenfalls eine Bewegung ausführt. Diese Bewegung kann das Schließen beispielsweise eines Schneidwerkzeuges bedeuten. Die Verschiebung des beweglichen Grundkörpers 20 die durch das Schließen der Griffstücke 27,27' am Bedienelement 4 erfolgt, führt in Richtung des proximalen Endes des Bedienelements 4 und zum Scheitelpunkt 25 des Pinzettenelements 22. Zwischen dem Scheitelpunkt 25 und dem Grundkörper 20 befindet sich in der geöffneten Stellung der Griffstücke 27,27' ein Hohlraum 33. Dieser Hohlraum 33 nimmt beim Schließen des Bedienelements 4 den axial verschiebbaren Grundkörper 20 auf. Das Öffnen der Griffstücke 27,27' aus der geschlossenen Position (Fig.1) in die geöffnete Position (Fig.2) erfolgt durch die Betätigung des erfindungsgemäßen integrierten Verriegelungsmechanismus 35. Nähere Ausführungen zum Verriegelungsmechanismus 35 kann der Fig.3 entnommen werden. Beim Öffnen der Griffstücke 27,27' ist fast keine Kraftaufwendung durch den Bediener erforderlich. Die Kraft zum Öffnen der Griffstücke 27,27' und somit das Verschieben des Grundkörpers 20 in axialer Richtung, erfolgt erfindungsgemäß durch die in den gefederten Schenkeln 23,23' des Elements 22 enthaltene Federkraft. Eine zusätzliche angeordnete Druckfeder zwischen den beiden Griffstücken 27 oder zwischen den Griffstücken 27,27' und dem Grundkörper 20 ist nicht erforderlich. Die Verschiebung des beweglichen Grundkörpers 20 hat zur Folge, dass das über die Betätigungsstange 21 und dem Zug- und Druckelement 5, am Ende des distalen Instrumentenschaftes 2, angeschossene Arbeitselement 3 ebenfalls eine Bewegung ausführt. Diese Bewegung kann das Öffnen beispielsweise eines Schneidwerkzeuges bedeuten. Die Verschiebung des beweglichen Grundkörpers 20, die durch das Öffnen der Griffstücke 27,27' am Bedienelement 4 erfolgt, führt in Richtung des distalen Endes des Instrumentenschaftes 2. Zwischen dem Scheitelpunkt 25 und dem verschiebbaren Grundkörper 20 entsteht wieder ein Hohlraum 33. Die Auslenkung der Griffstücke 27,27' in die geöffnete Position ist durch die Hebel 30,30' begrenzt.

Die Fig.3 zeigt in seitlicher Darstellung das geöffnete Bedienelement 4 im Schnitt mit angeschlossenen Instrumentenschaft 2 und dem erfindungsgemäßen Verriegelungsmechanismus 35. Der Verriegelungsmechanismus 35 weist im wesentlichen eine Riegelfeder 36, die als Blattfeder ausgebildet ist, einen Riegelarm 39 mit Riegelraste 42 und Befestigungsstift 37, und einem Verriegelungsstift 46 auf, wobei der Verriegelungsmechanismus 35 mit seinen Einzelteilen nicht sichtbar vom Bediener unterhalb der Griffschalen 26,26' in Ausnehmungen 47 der gefederten Schenkel 23,23' und im Durchbruch 48 des Grundkörpers 20 angeordnet ist. Die Blattfeder 36 entspricht in ihrer geometrischen Form einem flachen rechteckigen Körper, der mit seiner Unterseite auf der Oberfläche des gefederten Schenkel 23 uzw im Randbereich der Ausnehmung 47 aufliegt und somit die Ausnehmung 47 verschließt, während die Oberseite der Blattfeder 36 in eine an der Unterseite der Griffschale 26 angeordnete Vertiefung 49 eingreift. An der Unterseite der Blattfeder 36 befindet sich in deren symmetrischer Mitte ein Riegelarm 39, der einerseits senkrecht zu der Blattfeder 36 und mit seiner Stirnfläche an dieser befestigt ist und andererseits am oberen Ende 40 in der Nähe der Stirnfläche und parallel zu dieser an einem Befestigungsstift 37 angeordnet ist, wobei der Befestigungsstift 37 parallel und quer zur Blattfeder 36 beabstandet ist. Der Riegelarm 39 ist um den Befestigungsstift 37 drehbar gelagert, wobei die Elongation des Riegelarms 39 durch die Größe und Form der Ausnehmung 47 und der Bewegungsfreiheit der Blattfeder 36 begrenzt wird, d.h. die Auslenkung des Riegelarmes 39 erfolgt um den Riegeldrehpunkt 38 aus der Senkrechten zur Blattfeder 36. Der Riegelarm 39 selbst entspricht am oberen Ende 40 in seinen Abmessungen in etwa einer quadratischen Säule, die am unteren Ende 41 eine verjüngende Abstufung enthält, an der eine Riegelraste 42 angeordnet ist. In der geöffneten Position der Griffstücke 27,27' befindet sich der Riegelarm 39, mit seiner daran angeordneten Riegelraste 42, oberhalb des Verriegelungsstiftes 46, der in einer Ausnehmung 47' des gefederten Schenkel 23' angebracht ist und sich in senkrechter Stellung zu diesem befindet, wobei die Riegelraste 42 in der Seitenansicht geometrisch betrachtet, im weitesten Sinne einem spitzwinkligem Dreieck entspricht und als Körper einem dreiseitigem Prisma. Eine Seite des Prismas besitzt eine gerade Fläche 44, während die anderen beiden Seiten 43 des Prismas mit unterschiedlichen Radien an den Flächen ausgestattet sind. Die dem Verriegelungsstift 46 zugewandte Seite des Prismas entspricht der geraden Fläche 44 des Prismas, die einen Einrastweg 44 für den Verriegelungsstift 46 bildet, an dessen erster Fläche der Verriegelungsstift 46, beim Schließen der Griffstücke 27,27', entlang gleitet. Die Fläche des Einrastweges 44 steht nicht senkrecht zum Verriegelungsstift 46, sondern ist unter einem bestimmten Winkel zum Verriegelungsstift 44 geneigt angeordnet. Der Winkel, bzw. die Neigung des Einrastweges 44 zum Verriegelungsstift 46, bestimmt mit, die benötigte Kraft, die beim Schließen des Bedienelements 4 aufgebracht werden muss, um in die verriegelte Stellung bzw. geschlossene Position der Griffstücke 27,27 (siehe Fig.1) zu gelangen. Beim Schließen der axial angeordneten Griffstücke 27,27' gegen die Kraft der gefederten Schenkel 23,23', stößt die Fläche des Einrastweges 44 der Riegelraste 42 gegen den starren Verriegelungsstift 46. Damit die Riegelraste 42 am Verriegelungsstift 46 entlang gleiten kann, muss der Riegelarm 39 um seinen Riegeldrehpunkt 38 und gegen die Senkrechte der Blattfeder 36 ausschwenken, bis der Verriegelungsstift 46 am Wendepunkt I 51 angekommen ist, an dem sich die zweite angrenzende Fläche 53 des Prismas, eine nach Innen weisende Rastfläche 53, die eine Raststellung 45 beinhaltet, anschließt. Am Wendepunkt I 51, am Ende des Einrastweges 44, befindet sich die Riegelraste 42 mit dem Riegelarm 39 in der maximalen Auslenkung und der Verriegelungsstift 46 steht am Anfang der zweiten Fläche 53, wobei der Wendepunkt I 51 mit einem Radius ausgestattet ist. Da die zweite Fläche 53 ähnlich einem Innenradius ausgelegt ist, gleitet die Riegelraste 42, aufgrund der durch die Blattfeder 36 erzeugten Federkraft, bis zur Raststellung 45 in der Rastfläche 53 an dem starren Verriegelungsstift 46 entlang. D.h., die Federkraft der Blattfeder 36 versucht den aus gelenkten Riegelarm 39 in seine ursprüngliche senkrechte Stellung zurückzuholen. Befindet sich der Verriegelungsstift 46 in der Raststellung 45, liegen die Griffstücke 27,27' bis auf einen geringen Betrag am Grundkörper 20 an und die Betätigungsstange 21 ist in der zurückgezogenen Stellung und der Riegelarm 39 des Verriegelungsmechanismus 35 hat sich auf seiner Kreisbahn der Senkrechten der Blattfeder 36 angenähert. Die gelenkigen Hebel 30,30' zwischen dem Griffstück 27,27' und dem Grundkörper 20 sind ebenfalls eingefahren und in Öffnungen (nicht dargestellt) des Grundkörpers 20 eingetaucht. Das Bedienelement 4 ist geschlossen.

Das Öffnen des Bedienelements 4 erfolgt durch einen Druck auf die Griffstücke 27,27', beispielsweise erzeugt durch Zeigefinger und Daumen einer Hand des Bedieners. Durch den Druck auf die Griffstücke 27,27' werden diese um den noch verbliebenen geringen Betrag, den Abstand zwischen den Griffstücken 27,27' und dem Grundköper 20, aufeinander zu bewegt, wobei gleichzeitig die Riegelraste 42 die Raststellung 45 beim Verriegelungsstift 46 in Richtung des zweiten Wendepunkts II 52 verlässt. Die Riegelraste 42 gleitet dabei, aufgrund der von dem Benutzer erzeugten Kraft, weil der Riegelarm 39 noch aus der Senkrechten zur Blattfeder 36 ausgelenkt ist, an der zweiten Rastfläche bis zum zweiten Wendepunkt II 52 am starren Verriegelungsstift 46 entlang, wobei der Wendepunkt II 52 mit einem Radius ausgebildet ist. Ist die Riegelraste 42 am Wendepunkt II 52 angelangt, gleitet aufgrund der Federkraft der Blattfeder 36 die Riegelraste 42 mit der dritten Fläche 43, die dem Ausrastweg 43 entspricht, am Verriegelungsstift 46, entlang, wobei der Riegelarm in seine ursprüngliche senkrechte Stellung zurückschwenkt. Auf dem Ausrastweg 43 durchläuft der Riegelarm 39 auf der Kreisbahn, kurz nach dem Wendepunkt II 52, einmal die senkrechte Stellung und wird zur anderen Seite der Senkrechten ausgelenkt, bis der Ausrastweg 44 endet. Am Ende des Ausrastweges 44 schwenkt der Riegelarm 39 durch die Kraft der Blattfeder 36 in seine ursprüngliche Ausgangstellung zurückgeholt und die erste Fläche des Einrastweges 44 steht wieder oberhalb des Verriegelungsstiftes 46 zum nächsten Verriegelungsvorgang bereit. Der Verriegelungs- und Entriegelungsvorgang erfolgt automatisch und unterstützend für den Benutzer unsichtbar unter den Griffschalen 26,26', bzw. zwischen den Griffstücken 27,27'ab. Der weitere Öffnungsweg der am Bedienelement 4 angeordneten Griffstücke 27,27 erfolgt mit Hilfe der in den gefederten Schenkeln 23,23' noch verbleibenden Federkraft, bis der Endpunkt erreicht ist. Das Bedienelement 4 ist geöffnet und die Betätigungsstange befindet sich in vorgeschobener Stellung. Der Vorgang des Schließens des Bedienelements 4 und das automatische Verriegeln der Griffstücke 27,27', mit dem erfindungsgemäßen Verriegelungsmechanismus 35, kann jetzt wiederholt werden.

### Bezugszeichenliste:

- 1: Chirurgisches Instrument
- 2: Röhrenförmigen Element
- 3: Arbeitselement
- 4: Bedienelement
- 5: Zug- und Druckelement
- 6: Schaftrohr
- 7: Koppelelement
- 8: Kugel
- 9: Schaftverjüngung
- 10: Öffnung
- 11: Kupplung
- 12: Schlüsselloch
- 13: Instrumentschaftaufnahme
- 14: Außengewinde
- 15: Innengewinde
- 16: Überwurfmutter
- 17: Anschlussnippel
- 18: Bohrung
- 19: Einführschräge
- 20: Grundkörper
- 21: Betätigungsstange
- 22: Element
- 23,23': Pinzettenarme
- 24,24': Verjüngung
- 25: Scheitelpunkt
- 26,26': Griffschalen
- 27,27': Griffstücke
- 28,28': Befestigungsmittel
- 29,29': Benutzeroberfläche
- 30,30': Bauteile
- 31,31': Befestigungsstift
- 32: Befestigungsstift
- 33: Hohlraum
- 34:
- 35: Verriegelungsmechanismus
- 36: Riegelfeder
- 37: Befestigungsstift
- 38: Riegeldrehpunkt
- 39: Riegelarm
- 40: Oberes Ende
- 41: Unteres Ende
- 42: Riegelraste
- 43: Ausrastweg
- 44: Einrastweg
- 45: Raststellung
- 46: Verriegelungstift
- 47,47': Ausnehmungen
- 48: Durchbruch
- 49: Vertiefung
- 50: Unterseite
- 51: Wendepunkt
- 52: Wendepunkt II

## Patentansprüche

1. Medizinisches Instrument, insbesondere chirurgisches Instrument (1) zur Verwendung in der minimalinvasiven Chirurgie, mit einem röhrenförmigen Element (2), einem Arbeitselement (3) und einem axialen Bedienelement (4), welches lösbar am proximalen Ende des röhrenförmigen Elements (2) angeordnet ist und ferner mit einem in dem röhrenförmigen Element verschiebbar geführten Zug- und Druckelement (5), dessen vorderes Ende das Arbeitselement (3) betätigt und dessen rückwärtiges Ende über ein Koppelelement (7) lösbar mit dem beweglichen Bedienelement (4) verbunden ist, welches folgende Elemente aufweist
- es hat in geschlossenen Zustand die Form eines langges drechten Körpers und umfast einen beweglichen Grundkörper (20) mit einer Betädigungstange (21),
- es umfast ein Element (22), welches den Grundkörper (20) umschließende gefederte Schenkel (23,23') mit daran angeordneten, sich symmetrisch gegenübesliegenden griffstücken (27,27') aufweist
- es weist mindestens ein Bauteil (30,30') auf, welches den Grundkörper (20) mit den Griffstücken (23, 27) gelenkig verbindet,
- es umfast einen Verriegelungsmechanismus (35), der die beiden Griffstücke (27,27') ver- oder entriegelt, und
- es weist eine Instrumentenschafiaufnahme (13) auf welche die Betätigungsstange (21) des Grundkörpers (20) aufnimmt, und die eine Kupplung (11) mit einer Schlüsselloch (12) förmigen Öffnung (10,12) und einen Anschlussnippel (17),
umfasst.

2. Medizinisches Instrument, nach Anspruch 1, **dadurch gekennzeichnet, dass** der Verriegelungsmechanismus (35) im Bedienelement (4) integriert ist.

3. Medizinisches Instrument, nach Anspruch 1 bis 2, **dadurch gekennzeichnet, dass** der Verriegelungsmechanismus (35) automatisch durch die Bewegung der Griffstücke (27,27') das Bedienelement (4) ver- oder entriegelt.

4. Medizinisches Instrument, nach Anspruch 1 bis 3, **dadurch gekennzeichnet, dass** der Verriegelungsmechanismus (35) eine Riegelfeder (36), einen Riegelarm (39), eine Riegelraste (42) und einen Verriegelungsstift (46) umfasst.

5. Medizinisches Instrument, nach Anspruch 4, **dadurch gekennzeichnet, dass** die Riegelfeder (36), der Riegelarm (39) und die Riegelraste (39) miteinander zu einer Funktionseinheit verbunden sind.

6. Medizinisches Instrument, nach Anspruch 4 **dadurch gekennzeichnet, dass** die Riegelfeder (36) zwischen einer Griffschale (26) und einem gefederten Schenkel (23) angeordnet ist.

7. Medizinisches Instrument, nach Anspruch 4, **dadurch gekennzeichnet, dass** der an der Riegelfeder (36) angeordnete Riegelarm (39) einen Befestigungsstift (37) aufweist, um den der Riegelarm (39) drehbar gelagert ist, wobei der Befestigungsstift (37) einen Riegeldrehpunkt (38) bildet.

8. Medizinisches Instrument, nach einen der vorhergehenden Ansprüche, **dadurch gekennzeichnet, dass** die gefederten Schenkel (23,23') eine Ausnehmung (47,47') und der Grundkörper (20) einen Durchbruch (48) aufweist.

9. Medizinisches Instrument, nach Anspruch 4 **dadurch gekennzeichnet, dass** der Riegelarm (39) um den Riegeldrehpunkt (38) in der Ausnehmung (47,47') des gefederten Schenkel (23,23') und dem Durchbruch (48) im Grundkörper (20) nach rechts und links Ausschwenken kann.

10. Medizinisches Instrument, nach Anspruch 9, **dadurch gekennzeichnet, dass** die Ausschwenkung des Riegelarms (39) durch die Riegelraste (42) erfolgt, wenn diese am starren Verriegelungsstift (46), der in der Ausnehmung (47') des gefederten Schenkels (23') angeordnet ist, entlang gleitet.

11. Medizinisches Instrument, nach Anspruch 4, **dadurch gekennzeichnet, dass** die Riegelraste (42) zum entlang Gleiten am Verriegelungsstift (46) mit Gleitflächen ausgestattet ist.

12. Medizinisches Instrument, nach Anspruch 11, **dadurch gekennzeichnet, dass** die Gleitflächen einen Einrastweg (44), eine Raststellung (45) und eine Ausrastweg (43) bilden.

13. Medizinisches Instrument, nach Anspruch 4, **dadurch gekennzeichnet, dass** der Verriegelungsstift (46) in der Raststellung (45) das Bedienungselement (4) verriegelt.

14. Medizinisches Instrument, nach Anspruch 4, **dadurch gekennzeichnet, dass** beim Drücken auf die geschlossenen Griffstücke (27,27') die Riegelraste (42) den Verriegelungsstift (46) freigibt, wodurch die Griffstücke (27,27') sich daraufhin öffnen.

15. Medizinisches Instrument, nach einen der vorhergehenden Ansprüche, **dadurch gekennzeichnet, dass** die Griffschalen (26,26') ergonomisch gestaltet und an der Benutzeroberfläche strukturiert sind.

16. Medizinisches Instrument, nach einen der vorhergehenden Ansprüche, **dadurch gekennzeichnet, dass** der Anschlussnippel (17) zum Anschluss von Leitungen geeignet, durch die Flüssigkeiten gelangen können.

17. Medizinisches Instrument, nach einen der vorhergehenden Ansprüche, **dadurch gekennzeichnet, dass** die Kupplung (11) der Instrumentschaftaunahme (13) die Öffnung (10,12) enthält, die geeignet ist einen Kugelkopf eines Zug- und Druckelements (5) aufzunehmen.

18. Medizinisches Instrument, nach einen der vorhergehenden Ansprüche, **dadurch gekennzeichnet, dass** die Öffnung (10,12) der Kupplung (11) mit einer Einführschräge (19) ausgestattet ist.

## Claims

1. Medical instrument, in particular surgical instrument (1) for use in minimally invasive surgery, with a tubular element (2), a working element (3) and an axial operating element (4), which is arranged detachably at the proximal end of the tubular element (2), and with in addition, guided displaceably in the tubular element, a tension and compression element (5), the front end of which actuates the working element (3) and the rear end of which is detachably connected via a coupling element (7) with the movable operating element (4), which has the following elements:
it has, in the closed state
- the shape of an elongated cylindrical body and comprises a movable base body (20) with an actuating rod (21),
- it comprises an element (22), which has spring-loaded legs (23, 23'), surrounding the base body (20), with gripping pieces (27, 27') arranged thereon, lying opposite each other symmetrically,
- it has at least one component (30, 30'), which connects the base body (20) articulately with the gripping pieces (27, 27'),
- it comprises a locking mechanism (35), which locks or unlocks the two gripping pieces (27, 27'), and
- it has an instrument shaft receptacle (13), which receives the actuating rod (21) of the base body (20), and which comprises a coupling (11) with a keyhole-shaped opening (10, 12) and a connection nipple (17).

2. Medical instrument according to Claim 1, **characterized in that** the locking mechanism (35) is integrated in the operating element (4).

3. Medical instrument according to Claim 1 to 2, **characterized in that** the locking mechanism (35) automatically locks or unlocks the operating element (4) by the movement of the gripping pieces (27, 27').

4. Medical instrument according to Claim 1 to 3, **characterized in that** the locking mechanism (35) comprises an interlock spring (36), an interlock arm (39), an interlock retainer (42) and a locking pin (46).

5. Medical instrument according to Claim 4, **characterized in that** the interlock spring (36), the interlock arm (39) and the interlock retainer (39) are connected with each other to form a functional unit.

6. Medical instrument according to Claim 4, **characterized in that** the interlock spring (36) is arranged between a gripping shell (26) and a spring-loaded leg (23).

7. Medical instrument according to Claim 4, **characterized in that** the interlock arm (39), arranged on the interlock spring (36), has a fastening pin (37), about which the interlock arm (39) is rotatably mounted, wherein the fastening pin (37) forms an interlock pivot (38).

8. Medical instrument according to one of the preceding claims, **characterized in that** the spring-loaded legs (23, 23') have a recess (47, 47') and the base body (20) has an opening (48).

9. Medical instrument according to Claim 4, **characterized in that** the interlock arm (39) can swing out to the right and left about the interlock pivot (38) in the recess (47, 47') of the spring-loaded leg (23, 23') and the opening (48) in the base body (20).

10. Medical instrument according to Claim 9, **characterized in that** the swinging out of the interlock arm (39) takes place through the interlock retainer (42), when the latter slides along the rigid locking pin (46) which is arranged in the recess (47') of the spring-loaded leg (23').

11. Medical instrument according to Claim 4, **characterized in that** the interlock retainer (42) is equipped with sliding surfaces for sliding along the locking pin (46).

12. Medical instrument according to Claim 11, **characterized in that** the sliding surfaces form an engagement path (44), a detent position (45) and a disengagement path (43).

13. Medical instrument according to Claim 4, **characterized in that** the locking pin (46) locks the operating element (4) in the detent position (45).

14. Medical instrument according to Claim 4, **characterized in that** on pressing onto the closed gripping pieces (27, 27') the interlock retainer (42) releases the locking pin (46), whereby the gripping pieces (27, 27') subsequently open.

15. Medical instrument according to one of the preceding claims, **characterized in that** the gripping shells (26, 26') are ergonomically designed and are structured on the operator surface.

16. Medical instrument according to one of the preceding claims, **characterized in that** the connection nipple (17) is suitable for the connection of lines through which fluids can reach.

17. Medical instrument according to one of the preceding claims, **characterized in that** the coupling (11) of the instrument shaft receptacle (13) contains the opening (10, 12), which is suitable for receiving a ball head of a tension and compression element (5).

18. Medical instrument according to one of the preceding claims, **characterized in that** the opening (10, 12) of the coupling (11) is equipped with a lead-in chamfer (19).

## Revendications

1. Instrument médical, notamment instrument chirurgical (1) s'utilisant en chirurgie à invasion minimale, comportant un élément de forme tubulaire (2), un élément opérationnel (3) et un élément de manoeuvre axial (4), qui est disposé de manière amovible à l'extrémité proximale de l'élément de forme tubulaire (2), et comportant en outre un élément de traction et de pression (5) guidé de manière mobile dans l'élément de forme tubulaire et dont l'extrémité avant actionne l'élément opérationnel (3) et dont l'extrémité arrière est raccordée de manière amovible via un élément de couplage (7) à l'élément de manoeuvre mobile (4) et qui présente les éléments suivants:
à l'état fermé, il a
- la forme d'un corps cylindrique étiré en longueur et comprend un corps de base mobile (20) équipé d'une barre d'actionnement (21),
- il comprend un élément (22), qui présente des branches à suspension (23,23') entourant le corps de base (20) et où sont disposées des pièces de préhension (27, 27') se faisant face symétriquement,
- il présente au moins une composante (30,30') qui raccorde de manière articulée le corps de base (20) aux pièces de préhension (27,27'),
- il comprend un mécanisme de verrouillage (35) qui verrouille ou déverrouille les deux pièces de préhension (27,27'), et
- il présente un récepteur de tige d'instrument (13) qui reçoit la barre d'actionnement (21) du corps de base (20) et qui comprend un accouplement (11) pourvu d'un orifice en forme de trou de serrure (10, 12) et un raccord (17).

2. Instrument médical selon la revendication 1, **caractérisé en ce que** le mécanisme de verrouillage (35) est intégré dans l'élément de manoeuvre (4).

3. Instrument médical selon les revendications 1 à 2, **caractérisé en ce que** le mécanisme de verrouillage (35) verrouille ou déverrouille automatiquement l'élément de manoeuvre (4) par le mouvement des éléments de préhension (27,27').

4. Instrument médical selon les revendications 1 à 3, **caractérisé en ce que** le mécanisme de verrouillage (35) comprend un ressort de verrou (36), un bras de verrou (39), un cran de verrou (42) et une tige de verrouillage (46).

5. Instrument médical selon la revendication 4, **caractérisé en ce que** le ressort de verrou (36), le bras de verrou (39) et le cran de verrou (42) sont raccordés entre eux pour former une unité fonctionnelle.

6. Instrument médical selon la revendication 4, **caractérisé en ce que** le ressort de verrou (36) est disposé entre une coquille de préhension (26) et une branche à suspension (23).

7. Instrument médical selon la revendication 4, **caractérisé en ce que** le bras de verrou (39) disposé au niveau du ressort de verrou (36) présente une tige de fixation (37) autour de laquelle le bras de verrou (39) s'appuie en rotation, la tige de fixation (37) formant un point de rotation du verrou (38).

8. Instrument médical selon une des revendications précédentes, **caractérisé en ce que** les branches à suspension (23,23') présentent un évidement (47,47') et le corps de base (20) une percée (48).

9. Instrument médical selon la revendication 4, **caractérisé en ce que** le bras de verrou (39) peut pivoter vers la gauche et la droite autour du point de rotation du verrou (38) dans l'évidement (47,47') de la branche à suspension (23,23') et dans la percée (48) du corps de base (20).

10. Instrument médical selon la revendication 9, **caractérisé en ce que** le pivotement du bras de verrou (39) est déclenché par le cran de verrou (42) lorsque ce dernier glisse le long de la tige de verrouillage rigide (46) qui est disposée dans l'évidement (47') de la branche à suspension (23').

11. Instrument médical selon la revendication 4, **caractérisé en ce que** le cran de verrou (42) est pourvu de surfaces de glissement pour glisser le long de la tige de verrouillage (46).

12. Instrument médical selon la revendication 11, **caractérisé en ce que** les surfaces de glissement constituent une voie d'enclenchement (44), une position d'enclenchement (45) et une voie de désenclenchement (43).

13. Instrument médical selon la revendication 4, **caractérisé en ce que** la tige de verrouillage (46) verrouille l'élément de manoeuvre (4) dans la position d'enclenchement (45).

14. Instrument médical selon la revendication 4, **caractérisé en ce que**, en appuyant sur les pièces de préhension fermées (27,27'), le cran de verrou (42) libère la tige de verrouillage (46), ce qui a pour effet que les pièces de préhension (27,27') s'ouvrent alors.

15. Instrument médical selon une des revendications précédentes, **caractérisé en ce que** les coquilles de préhension (26,26') sont de forme ergonomique et structurées sur leur surface utilisateur.

16. Instrument médical selon une des revendications précédentes, **caractérisé en ce que** le raccord (17) convient pour raccorder des conduites par lesquelles des liquides peuvent arriver.

17. Instrument médical selon une des revendications précédentes, **caractérisé en ce que** l'accouplement (11) du récepteur de tige d'instrument (13) comprend l'orifice (10, 12) qui est apte à recevoir une tête sphérique d'un élément de traction et de pression (5).

18. Instrument médical selon une des revendications précédentes, **caractérisé en ce que** l'orifice (10, 12) de l'accouplement (11) est équipé d'une pente d'introduction (19).
